(19) Europäisches Patentamt

European Patent Office

Office européen des brevets

(11) Veröffentlichungsnummer: **0 343 261 B1**

(12) **EUROPÄISCHE PATENTSCHRIFT**

(45) Veröffentlichungstag der Patentschrift: **12.08.92**

(21) Anmeldenummer: **88107995.8**

(22) Anmeldetag: **19.05.88**

(51) Int. Cl.⁵: **G01N 13/00**, G01N 33/15, G01N 35/06

(54) **Dissolutionstestgerät.**

(43) Veröffentlichungstag der Anmeldung:
**29.11.89 Patentblatt 89/48**

(45) Bekanntmachung des Hinweises auf die
Patenterteilung:
**12.08.92 Patentblatt 92/33**

(84) Benannte Vertragsstaaten:
**BE DE ES FR GB IT NL**

(56) Entgegenhaltungen:
**EP-A- 0 121 345      EP-A- 0 210 014
EP-A- 0 246 632      DE-U- 8 336 233
FR-A- 2 597 607      US-E- 27 637**

(73) Patentinhaber: **ERWEKA GmbH
Ottostrasse 20-22
W-6056 Heusenstamm(DE)**

(72) Erfinder: **Schneider, Ortwin
Arheilgerstrasse 39
W-6108 Weiterstadt(DE)**

(74) Vertreter: **Patentanwälte Kirschner & Grosse
Forstenrieder Allee 59
W-8000 München 71(DE)**

Rank Xerox (UK) Business Services

## Beschreibung

Die vorliegende Erfindung betrifft ein Dissolutionstestgerät nach dem Oberbegriff des Patentanspruchs 1. Insbesondere befaßt sich die vorliegende Erfindung mit einem Dissolutionstestgerät, bei dem zwischen verschiedenen Meßzyklen ein Wechseln von Filtern zumindest erleichtert wird und bei einem bevorzugten Ausführungsbeispiel vollautomatisch vorgenommen wird.

Ein gattungsgemäßes Dissolutionstestgerät wird von der Anmelderin gefertigt und vertrieben. Ein derartiges Dissolutionstestgerät dient zur Untersuchung des Auflösungsverhaltens eines Stoffes in einer Flüssigkeit und insbesondere zur Untersuchung des Auflösungsverhaltens einer Arzneimitteltablette in einer Flüssigkeit, deren Zusammensetzung im wesentlichen derjenigen der Magensäure entspricht.

Das bekannte Dissolutionstestgerät hat einen Spülstand, einen Teststand und eine bewegliche Vorrichtung, die in einem Wasserbad eine Mehrzahl von Meßbehältern hält. Der Spülstand hat für jeden Meßbehälter ein Spülpaddel, das in Drehrichtung antreibbar ist und in vertikaler Richtung anhebbar und absenkbar angeordnet ist. Der Teststand hat für jeden Meßbehälter ein Paddel zum Umrühren einer Meßflüssigkeit, wobei gleichfalls sämtliche Paddel in Drehrichtung antreibbar sind und zum Umrühren der Flüssigkeit in den Behältern, die den im Hinblick auf sein Auflösungsverhalten zu untersuchenden Stoff enthalten, in die Meßbehälter absenkbar sind. Ferner ist bei dem bekannten Dissolutionstestgerät für jeden Meßbehälter ein Absaugrohr vorgesehen, das zusammen mit dem vorgesehenen Paddel in die den untersuchenden Stoff enthaltende Flüssigkeit eintauchbar ist. Die bewegliche Vorrichtung, die die im Wasserbad angeordneten Meßbehälter aufweist, kann in horizontaler Richtung an den Ort des Spülstandes verfahren werden, wobei in dieser Lage der beweglichen Vorrichtung ein Absenken der Spülpaddel zum Zwecke des Spülens der Meßbehälter vorgenommen wird. Nach dem Abreinigen der Meßbehälter werden die Spülpaddel angehoben, so daß die bewegliche Vorrichtung zum Ort des Teststandes zurückgefahren werden kann. Wenn dieser Ort erreicht ist, können die Paddel in die Meßbehälter abgesenkt werden, um eine Flüssigkeit, in die zu einem bestimmten Zeitpunkt der zu untersuchende Stoff eingebracht wird, gleichmäßig umzurühren. Nach einer bestimmten Umrührzeit wird die Flüssigkeit mittels der Absaugrohre aus den Meßbehältern abgesaugt. Nach einem derartigen Zyklus ist es erforderlich, die Filter an den Spitzen der Absaugrohre gegen neue Filter auszutauschen, was einerseits arbeitsaufwendig ist und andererseits zumindest in einem gewissen Umfang eingewiesenes und geschultes Personal erfordert.

Gegenüber diesem Stand der Technik liegt der vorliegenden Erfindung die Aufgabe zugrunde, ein Dissolutionstestgerät der eingangs genannten Art so weiterzubilden, daß der Filterwechsel vollautomatisch durchgeführt werden kann.

Diese Aufgabe wird bei einem Dissolutionstestgerät nach dem Oberbegriff des Patentanspruchs 1 durch die im kennzeichnenden Teil des Patentanspruchs 1 angegebenen Merkmale gelöst.

Der Erfindung liegt die Erkenntnis zugrunde, daß die bei dem bekannten Dissolutionstestgerät erforderliche zyklische Bewegung der beweglichen Vorrichtung bei jedem Meßzyklus dazu ausgenutzt werden kann, den Filterwechsel zu automatisieren. An der beweglichen Vorrichtung ist eine sich mit der beweglichen Vorrichtung gemeinsam bewegende Filterabstreifvorrichtung angeordnet, mit der in einer bestimmten Lage der beweglichen Vorrichtung der Filter in Eingriff nehmbar ist.

Gemäß der bevorzugten Ausgestaltung nach Patentanspruch 2 ist diese Filterabstreifvorrichtung derart angeordnet, daß zunächst nach einer Bewegung der beweglichen Vorrichtung über einen Teil ihres Weges von dem Teststand zu dem Spülstand in die erste Lage der erste Filter in Eingriff nehmbar ist, woraufhin ein anschließendes Anheben des Absaugrohres zu einem Abziehen des Filters von dem Absaugrohr führt. Mit anderen Worten kann allein unter Ausnutzung der auch im Stand der Technik erforderlichen Horizontalbewegung der beweglichen Vorrichtung sowie der Vertikalbewegung des Absaugrohres ein automatisches Abstreifen der Filter von den Absaugrohren erzielt werden, ohne daß es hierzu eines nennenswerten steuerungstechnischen und apparativen Aufwandes bedürfte.

Bei der in Anspruch 3 festgelegten Ausgestaltung der Filteraufsetzvorrichtung als Drehscheibe, auf der in gleichmäßigen Winkelabständen eine Mehrzahl von neuen Filtern angeordnet ist, die durch eine Antriebsvorrichtung nach jedem Aufbringen eines neuen Filters auf das Absaugrohr um den Winkelabstand weiterdrehbar ist, kann eine Reihe von Filterwechseln vollautomatisch durchgeführt werden, ohne daß es hierzu einer Bedienungsperson bedarf. Besonders einfach kann das Weiterdrehen der Drehscheibe um die gleichmäßigen Winkelabstände dadurch durchgeführt werden, daß die Antriebsvorrichtung der Drehscheibe als Schrittmotor ausgebildet ist.

Ein besonders geringer baulicher Aufwand für eine Filteraufsetzvorrichtung wird gemäß Anspruch 5 dadurch erreicht, daß zumindest zwei Meßbehälter, Paddel, Spülpaddel und Absaugrohre jeweils nebeneinander in einer Richtung senkrecht zur Bewegungsrichtung der beweglichen Vorrichtung angeordnet sind und daß eine Drehscheibe als gemeinsame Filteraufsetzvorrichtung für zwei benachbarte Absaugrohre dient und derart angeordnet ist, daß die abgesenkten Absaugrohre in der zweiten Lage der beweglichen Vorrichtung mit zwei neuen Filtern Eingriff nehmen, die auf der Drehscheibe diametral gegenüberliegend angeordnet sind.

Besonders einfach kann das Absenken und Anheben der Absaugrohre zum Zwecke des Filterwechsels dadurch ausgeführt werden, daß die Absaugrohr zusammen mit den Paddeln an einer in vertikaler Richtung anhebbaren und absenkbaren Brücke angeordnet sind, wie dies in Patentanspruch 6 festgelegt ist.

Vorzugsweise ist gemäß Patentanspruch 7 ein Spülbehälter an der beweglichen Vorrichtung angeordnet, in den das Paddel in der ersten Lage der beweglichen Vorrichtung eintauchbar ist. Daher kann eine einzige Vorschublage der beweglichen Vorrichtung sowohl zum Abreinigen der Paddel als auch zum Ineingriffnehmen der Filter vor dem Filterentfernen ausgenutzt werden.

Zwei bevorzugte Ausführungsformen der vorliegenden Erfindung werden nachfolgend unter Bezugnahme auf die beiliegenden Zeichnungen näher erläutert. Es zeigen:

Fig. 1    eine Vorderansicht einer ersten Ausführungsform eines Dissolutionstestgerätes;

Fig. 2    eine Seitenansicht der in Fig. 1 gezeigten ersten Ausführungsform;

Fig. 3    ein Detail von Fig. 2, das in dieser Figur mit einem strichpunktierten Kreis hervorgehoben ist, der mit dem Bezugzeichen III bezeichnet ist;

Fig. 4    eine Draufsicht auf einen Ausschnitt einer zweiten, vollautomatischen Ausführungsform des Dissolutionstestgerätes; und

Fig. 5    eine Schnittansicht durch die in Fig. 4 gezeigte, zweite Ausführungsform längs der Linie V-V.

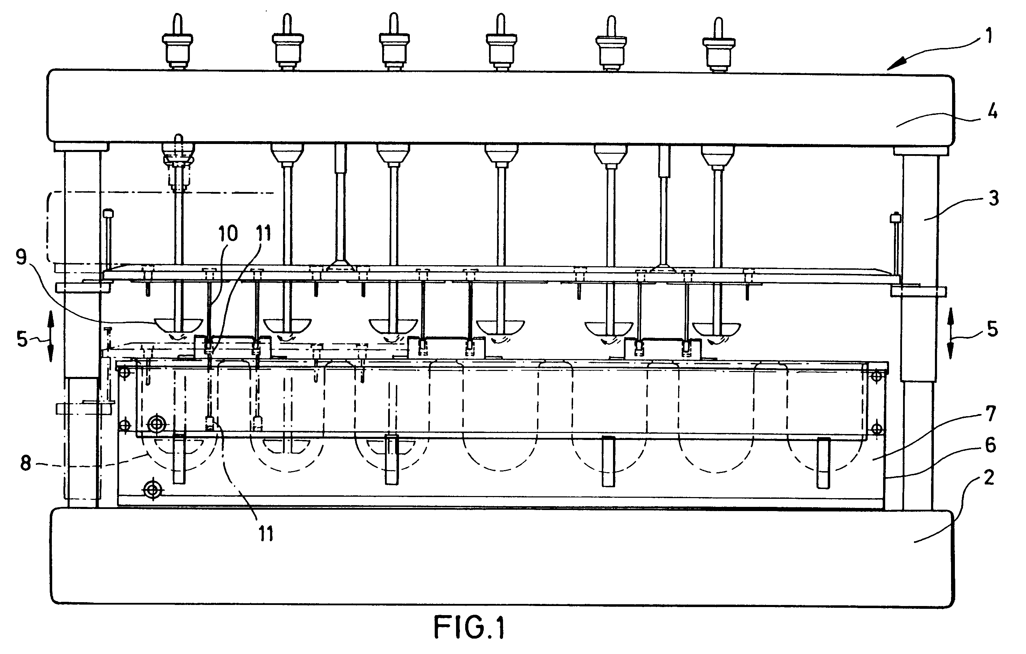

Wie in Fig. 1 gezeigt ist, umfaßt ein Dissolutionstestgerät 1 ein Bodenteil 2, in dem (nicht dargestellte) Antriebseinheiten untergebracht sind. Eine Brücke 4 ist über Säulen 3 gegenüber dem Bodenteil 2 höhenbeweglich angeordnet. Die Vertikalbeweglichkeit der Brücke 4 ist durch Pfeile 5 angedeutet. Oberhalb des Bodenteiles 2 ist eine bewegliche Vorrichtung 6 angeordnet, die in einer horizontalen, senkrecht zur Zeichenebene der Fig. 1 liegenden Bewegungsrichtung hin- und herverfahrbar ist. Die bewegliche Vorrichtung 6 umfaßt ein Wasserbad 7, das zur gleichmäßigen und genau festlegbaren Temperierung von Meßbehältern 8 dient. Die Meßbehälter sind zur Aufnahme einer Flüssigkeit vorgesehen, in der ein im Hinblick auf sein Auflösungsverhalten zu untersuchender Stoff auflösbar ist. Die Relativbewegung zwischen der Vorrichtung 6 einerseits und einem Teststand 13 und einem Spülstand 14 andererseits kann auch durch Verfahren von Teststand und Spülstand gegenüber der dann ortsfesten Vorrichtung 6 erfolgen.

An der Brücke 4 sind Paddel 9 gelagert, die durch eine (nicht dargestellte) Antriebsvorrichtung in Drehrichtung antreibbar sind. Ebenfalls sind mit der Brücke 4 Absaugrohre 10 verbunden, die an ihrem unteren Ende ein Filter 11 tragen.

Die Brücke 4, die an der Brücke gelagerten Paddel 9 sowie die mit der Brücke verbundenen Absaugrohre 10 sind in Fig. 1 in ihrer oberen vertikalen Endlage mit durchgezogenen Linien dargestellt und ihrer unteren vertikalen Endlage mit gestrichelten Linien angedeutet.

Wie man erkennen kann, tauchen die Paddel 9 in der unteren vertikalen Endlage der Brücke 4 in die Meßbehälter 8 ein. In diesem Zustand befinden sich die Filter 11 an den Enden der Absaugrohre 10 im unteren Bereich der Meßbehälter 8.

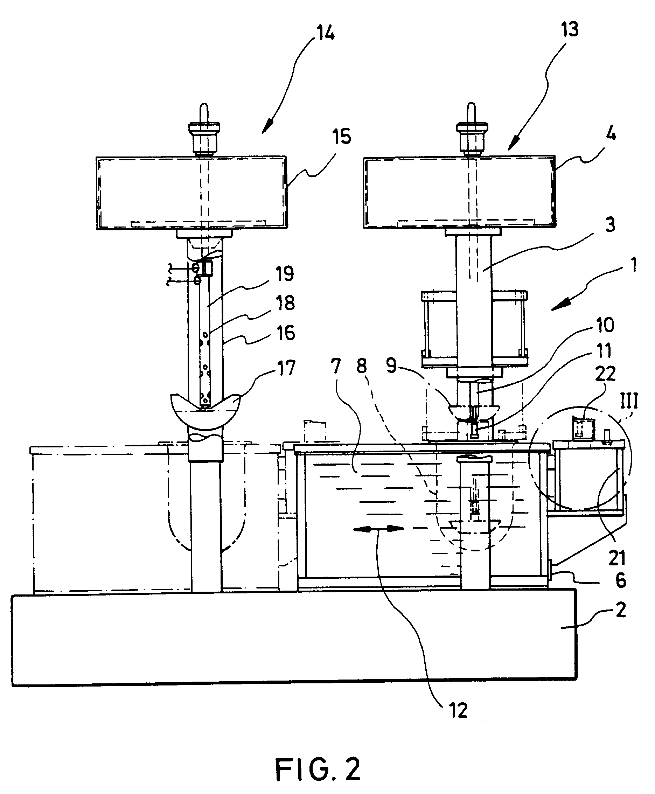

Fig. 2 zeigt eine Seitenansicht, teilweise in Querschnittsdarstellung, des in Fig. 1 gezeigten Dissolutionstestgerätes 1. Mit den in Fig. 1 verwendeten Bezugszeichen übereinstimmende Bezugszeichen bezeichnen gleiche Teile.

Die bewegliche Vorrichtung 6 ist gegenüber dem Bodenteil 2 in Richtung des Pfeiles 12 hin- und herverfahrbar. Im rechtsseitigen Bereich des Bodenteiles 2 ist ein Teststand 13 angeordnet, der im wesentlichen aus den Säulen 3, der Brücke 4, den Paddeln 9 und den Absaugrohren 10 gebildet wird und in Fig. 1 in Seitenansicht gezeigt ist.

Im linksseitigen Bereich des Bodenteiles 2 liegt ein Spülstand 14, der eine Brücke 15 aufweist, die über Säulen 16 gegenüber dem Bodenteil 2 in vertikaler Richtung beweglich ist. An der Brücke 15 des Spülstandes 14 sind Spülpaddel 17 drehbar gelagert. Die Spülpaddel 17 werden in Drehrichtung von einer (nicht dargestellten) Antriebseinheit angetrieben. Die Spülpaddel 17 haben einen hohlen Schaft 19, der mit Spüldüsen 18 versehen ist. Eine Spülflüssigkeit kann dem hohlen Schaft zugeführt werden und über die Düsen 18 in der abgesenkten Lage der Spülpaddel 17 zur Reinigung der Meßbehälter 8 ausgesprüht werden, wenn sich die bewegliche Vorrichtung 6 in der strichpunktiert gezeichneten, linksseitigen Lage zum Spülen der Meßbehälter 8 befindet.

Die bewegliche Vorrichtung 6 trägt an ihrem in Fig. 2 rechtsseitigen Ende einen Spülbehälter 21, in den das Paddel 9 in einer ersten Lage der beweglichen Vorrichtung 6 absenkbar ist. Diese erste Lage der beweglichen Vorrichtung 6 befindet sich zwischen der mit durchgezogenen Linien dargestellten Lage der beweglichen Vorrichtung 6 am Ort des Teststandes und der mit gestrichelten Linien dargestellten Lage der beweglichen Vorrichtung 6 am Ort des Spülstandes 14. In dieser ersten Lage nimmt eine gabelförmige Filterabstreifvorrichtung 22 Eingriff mit dem Filter 11 am unteren Ende des Absaugrohres 10, wobei sich das Absaugrohr 10 zusammen mit der Brücke 4 des Teststandes 13 in seiner vertikal angehobenen Lage befindet.

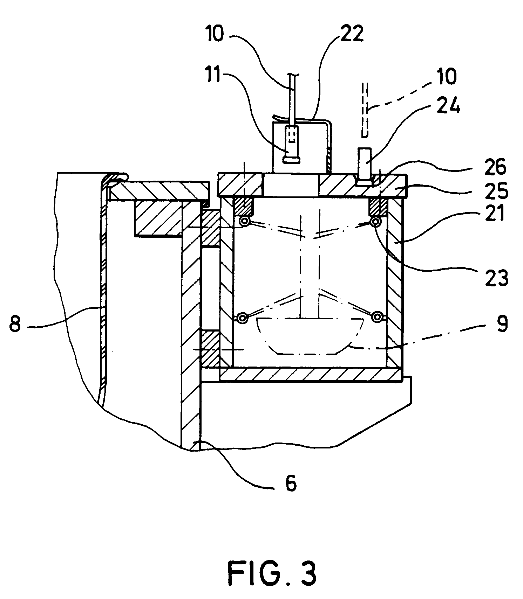

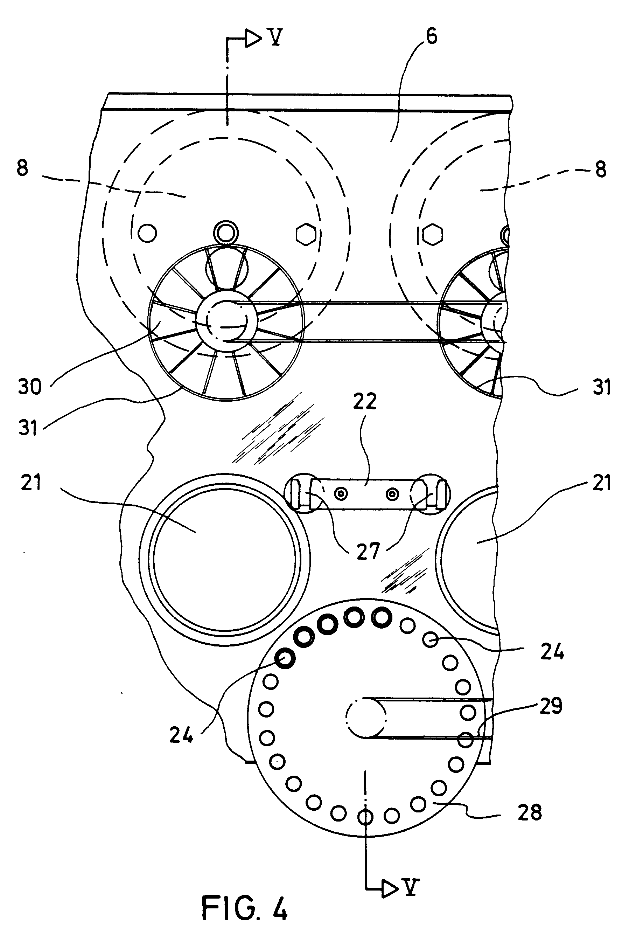

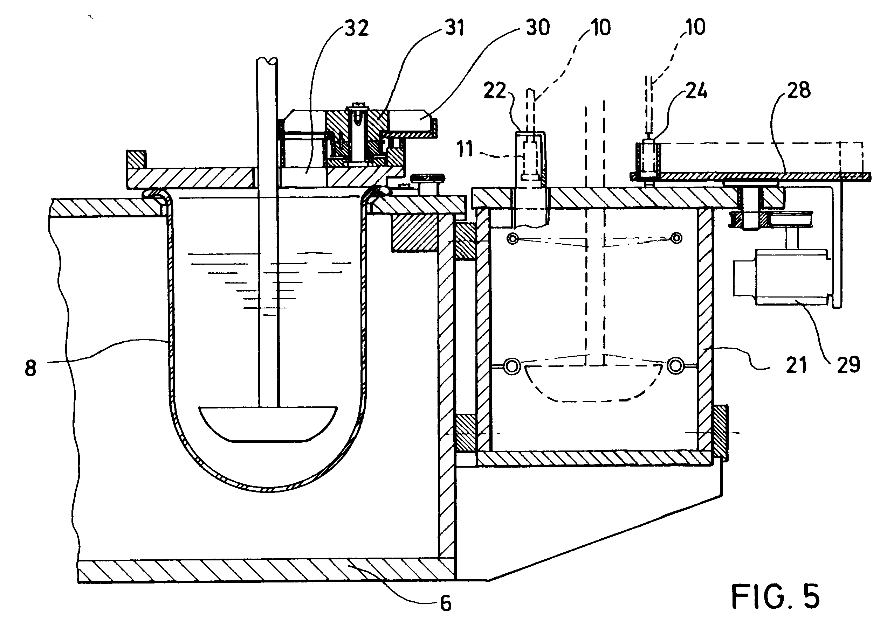

Das durch den strichpunktierten Kreis III in Fig. 2 hervorgehobene Detail der beweglichen Vorrichtung 6 ist in Fig. 3 vergrößert wiedergegeben. Die horizontale Lage der beweglichen Vorrichtung 6, in der sich diese bei der Darstellung gemäß Fig. 3 befindet, entspricht der oben definierten ersten Lage. Wie in Fig. 3 zu erkennen ist, greift die gabelförmige Filterabstreifvorrichtung 22 in dieser ersten Lage der beweglichen Vorrichtung 6 über den Filter 11 am Ende des Absaugrohres 10, das sich, wie bereits erläutert, hierbei in seiner in vertikaler Richtung zumindest teilweise angehobenen Lage befindet. Ein Anheben der Brücke 4 und damit des Absaugrohres 10 in dieser ersten Lage der beweglichen Vorrichtung 6 führt zu einem Abstreifen des Filters 11 von dem unteren Ende des Absaugrohres 10, woraufhin der Filter 11 durch eine Öffnung, die in den später zu erläuternden Fig. 4 und 5 besser zu erkennen ist, nach unten fällt. Anschließend wird die Brücke 4 abgesenkt, wodurch das Paddel 9 in die in Fig. 3 gezeigte, strichpunktierte Lage gebracht wird, in der es in dem Spülbehälter 21 mittels Reinigungsdüsen 23 von Rückständen der Flüssigkeit und des zu untersuchenden Stoffes aus dem vorherigen Testzyklus gereinigt wird.

Bei einem geringfügigen weiteren Verfahren der beweglichen Vorrichtung 6 in Richtung auf den Spülstand (in Fig. 3 nach links) erreicht das Absaugrohr 10 gegenüber der beweglichen Vorrichtung 6 die in Fig. 3 strichpunktiert gezeichnete Lage, in der das Absaugrohr 10 oberhalb eines neuen Filters 24 zu stehen kommt, der in einer Ausnehmung 26 des Deckels 25 des Spülbehälters 21 gehalten wird. Bei einem Absenken der Brücke 4 in dieser relativen Lage des Absaugrohres 10, die als zweite Lage bezeichnet wird, nimmt das Absaugrohr 10 mit seinem Ende Eingriff mit dem neuen Filter 24, der durch einen Klemmsitz oder Paßsitz auf dem Absaugrohr 10 durch einfaches Aufschieben festgelegt wird.

Bei dem unter Bezugnahme auf die Fig. 1 bis 3 erläuterten ersten Ausführungsbeispiel des erfindungsgemäßen Dissolutionstestgerätes kann durch Ausnutzung der Horizontalbewegung der beweglichen Vorrichtung 6 und der Vertikalbewegung der Brücke 4 ein Filter 11, der in einem vorherigen Meßzyklus zum Einsatz gekommen ist, von dem Absaugrohr 10 abgestreift und durch einen neuen Filter 24 für den nächsten Meßzyklus ersetzt werden. Jedoch ist es bei dieser Ausführungsform erforderlich, im Verlaufe des nun folgenden Meßzyklus einen weiteren, neuen Filter 24 in die Ausnehmung 26 des Deckels 25 zu legen.

Demgegenüber ermöglicht die nachfolgend beschriebene, in den Fig. 4 und 5 gezeigte zweite Ausführungsform einen vielfachen Filterwechsel, ohne daß es nach jedem Meßzyklus eines Eingreifens der Bedienungsperson bedarf.

In Fig. 4 ist eine Draufsicht auf einen Ausschnitt der zweiten Ausführungsform des Dissolutionstestgerätes gezeigt. In dieser Figur wie auch in der Fig. 5 bezeichnen wiederum solche Bezugszeichen, die unter Bezugnahme auf das erste Ausführungsbeispiel verwendet wurden, gleiche oder ähnliche Teile. Wie in der Draufsicht zu erkennen ist, liegt eine Filterabstreifvorrichtung 22 mit zwei gabelförmigen Ausnehmungen, in die zwei Absaugrohre 10 einschiebbar sind, zwischen zwei Spülbehältern 21. Die beiden gezeigten Spülbehälter 21 aus einer Mehrzahl von nebeneinander angeordneten Spülbehältern und die beiden Meßbehälter 8 aus einer Mehrzahl von nebeneinander angeordneten Meßbehältern sind jeweils senkrecht zu der Bewegungsrichtung der beweglichen Vorrichtung 6 nebeneinander angeordnet. In der Mitte zwischen jeweils zwei Spülbehältern 21 liegt eine Drehscheibe 28, auf der in gleichmäßigen Winkelabständen eine Vielzahl von Filtern 24 angeordnet ist. Die Drehscheibe 28 wird gemeinsam mit weiteren (nicht gezeigten) Drehscheiben über eine Antriebsverbindung 29 von einer durch einen Schrittmotor gebildeten Antriebsvorrichtung angetrieben.

Wie insbesondere in der Querschnittsdarstellung gemäß Fig. 5 zu sehen, wird die bewegliche Vorrichtung 6 zunächst in die erste Lage gefahren, in der sie die Relativlage gegenüber den Absaugrohren 10 einnimmt, bei der die Absaugrohre 10 unter der Filterabstreifvorrichtung liegen. In dieser Lage genügt eine Vertikalbewegung der Brücke 4 und somit der Absaugrohre 10 zum Abstreifen der Filter 11. Anschließend verfährt die bewegliche Vorrichtung in die zweite relative Lage zu den Absaugrohren 10, bei der die Absaugrohre 10 über den Filtern 24 liegen. Durch Absenken der Ansaugrohre 10 gegenüber der Drehscheibe 28 werden neue Filter 24 über die Absaugrohre 10 geschoben.

Im Verlauf des anschließenden Meßzyklus wird die Drehscheibe 28 um einen solchen Drehwinkel weiterbewegt, daß neue Filter 24 in der in Fig. 5 dargestellten Lage zu liegen kommen.

Wie in den Fig. 4 und 5 gleichfalls zu erkennen ist, sind oberhalb der Meßbehälter 8 mit sektorförmigen Aufnahmen 30 für die im Hinblick auf ihr Auflösungsvermögen zu untersuchenden Arzneimitteltabletten versehene Fördereinrichtungen 31 angeordnet, die in Drehrichtung schrittweise antreibbar sind. Wenn sich eine sektorförmige Aufnahme 30 über die Öffnung 32 im Boden der Fördereinrichtung 31 bewegt, fällt die zu untersuchende Arzneimitteltablette in den mit der Flüssigkeit gefüllten Meßbehälter 8. Durch diese Maßnahme kann ein zeitlich genau bestimmbarer Beginn des Disolutionstestes festgelegt werden.

In Abweichung zu der in den Fig. 4 und 5 gezeigten, drehscheibenförmigen Vorrichtung zum Aufnehmen von neuen Filtern und zum Befördern der neuen Filter in eine Lage, in der diese über die Absaugrohre schiebbar sind, kann auch eine lineare Aufnahme für Filter verwendet werden, die die Filter in einer linearen Richtung in die Lage bewegt, in der sie über die Absaugrohre schiebbar sind.

Bei Verwenden von winkelmäßig gegenüber der Horizontalen angestellten Filterabstreifvorrichtungen kann das Abstreifen der Filter 11 allein durch die Horizontalbewegung der beweglichen Vorrichtung 6 vorgenommen werden, ohne daß es zum Abstreifen eines Anhebens der Absaugrohre gegenüber der beweglichen Vorrichtung bedürfte.

**Patentansprüche**

1.  Dissolutionstestgerät (1)
    - mit einem Spülstand (14), der zumindest ein drehbar antreibbares und in vertikaler Richtung relativ zu einem Meßbehälter (8), anhebbares und absenkbares Spülpaddel (17) aufweist,
    - mit einem Teststand (13), der zumindest ein drehbar antreibbares und in vertikaler Richtung relativ zu dem Meßbehälter (8) anhebbares und absenkbares Paddel (9) aufweist,
    - mit einer Vorrichtung (6), die wenigstens einen Meßbehälter (8) aufweist, in dem mittels des abgesenkten Paddels (9) eine Flüssigkeit, die einen im Hinblick auf sein Auflösungsverhalten zu untersuchenden Stoff enthält, gerührt werden kann, und die zwischen dem Spülstand (14) und dem Teststand (13) im angehobenen Zustand des Paddels (9) und des Spülpaddels (17) verfahrbar ist,
    - wobei die Vorrichtung (6) mit dem oder den Meßbehälter (n) einerseits und Spülstand (14) und Teststand (13) andererseits horizontal gegeneinander verfahrbar sind,
    - und mit einem einen Filter (11) aufweisenden Absaugrohr (10), mit dem die den in Hinblick auf sein Auflösungsverhalten zu untersuchenden Stoff enthaltende Flüssigkeit aus dem Meßbehälter (8) absaugbar ist, wenn sich die bewegliche Vorrichtung am Ort des Teststandes (13) befindet, wobei das Absaugrohr (10) gemeinsam mit dem Anheben des Spülpaddels (17) und/oder des Paddels (9) relativ zu dem Meßbehälter (8) anhebbar und absenkbar ist,
    dadurch gekennzeichnet,
    - daß eine Filterabstreifvorrichtung (22) an der beweglichen Vorrichtung (6) angeordnet ist, mit der (22) in einer ersten Lage der beweglichen Vorrichtung (6) der Filter (11) in Eingriff nehmbar ist,
    - daß die Filterabstreifvorrichtung (22) derart angeordnet und ausgebildet ist,
        - daß bei einem anschließenden Anheben des Absaugrohres (10) der Filter (11) von dem Absaugrohr (10) abstreifbar ist,
    - daß eine Filteraufsetzvorrichtung (25, 26; 28) mit Aufnahmen für neue Filter (24) an der beweglichen Vorrichtung (6) angeordnet ist, und
    - daß das Absaugrohr (10) nach einer Bewegung der beweglichen Vorrichtung (6) in eine zweite Lage absenkbar ist und im abgesenkten Zustand mit dem neuen Filter (24) mittels Klemmsitz verbindbar ist.

2.  Dissolutionstestgerät nach Anspruch 1,
    dadurch gekennzeichnet,
    - daß die Filterabstreifvorrichtung (22) derart angeordnet ist,
        - daß zunächst nach einer Bewegung der beweglichen Vorrichtung (6) über einen Teil ihres Weges von dem Teststand (13) zu dem Spülstand (14) in die erste Lage der Filter (11) in Eingriff nehmbar ist und
        - daß bei einem anschließenden Anheben des Absaugrohres (10) der Filter (11) von dem Absaugrohr (10) abstreifbar ist.

3.  Dissolutionstestgerät nach Anspruch 1 oder 2,
    dadurch gekennzeichnet,
    daß die Filteraufsetzvorrichtung als Drehscheibe (28) ausgebildet ist, auf der in gleichmäßigen Winkelabständen eine Mehrzahl von neuen Filter (24) angeordnet ist, und
    daß die Drehscheibe (28) durch eine Antriebsvorrichtung (29) nach jedem Aufbringen eines neuen Filters (24) auf das Absaugrohr (10) um den Winkelabstand weiterdrehbar ist.

4.  Dissolutionstestgerät nach Anspruch 3,
    dadurch gekennzeichnet,
    daß die Antriebsvorrichtung ein Schrittmotor ist.

5.  Dissolutionstestgerät nach Anspruch 3 oder 4,
    dadurch gekennzeichnet,
    daß zumindest zwei Meßbehälter (8), zwei Paddel (9), zwei Spülpaddel (17) und zwei Absaugrohre (10) jeweils nebeneinander in einer Richtung senkrecht zu der Bewegungsrichtung der beweglichen Vorrichtung (6) angeordnet sind, und

daß die Drehscheibe (28) als gemeinsame Filteraufsetzvorrichtung für zwei benachbarte Absaugrohre (10) dient und derart angeordnet ist, daß die abgesenkten Absaugrohre (10) in der zweiten Lage der beweglichen Vorrichtung (6) mit zwei neuen Filtern (24) Eingriff nehmen, die auf dieser gemeinsamen Drehscheibe (28) angeordnet sind.

6. Dissolutionstestgerät nach einem der Ansprüche 1 bis 4,
dadurch gekennzeichnet,
daß eine in vertikaler Richtung anhebbare und absenkbare Brücke (4) vorgesehen ist, an der sowohl das Paddel (9) als auch das Absaugrohr (10) angeordnet sind.

7. Dissolutionstestgerät nach einem der Ansprüche 1 bis 6,
dadurch gekennzeichnet,
daß die bewegliche Vorrichtung (6) einen Spülbehälter (21) aufweist, in den das Paddel (9) in der ersten Lage der beweglichen Vorrichtung (6) eintauchbar ist.

**Claims**

1. Dissolution testing apparatus (1) comprising
   - a rinsing stand (14) comprising at least one rotatably drivable rinsing paddel (17) being liftable and lowerable in vertical direction relative to a measuring vessel (8),
   - a test stand (13) comprising at least one rotatably drivable paddle (9) being liftable and lowerable in vertical direction relative to the measuring vessel (8),
   - a means (6) comprising at least one measuring vessel (8), wherein by means of the lowered paddle (9) a liquid containing a substance to be examined with respect to its dissolution behaviour can be stirred, and said means (6) being reciprocable between the rinsing stand (14) and the test stand (13) during lifted position of the paddle (9) and the rinsing paddle (17),
   - wherein the means (6) with the one or several measuring vessel/s on one hand and said rinsing stand (3) and said test stand (13) on the other hand are horizontally shiftable with respect to one another,

   - and a suction pipe (10) comprising a filter (11), by means of which suction pipe (10) the liquid containing the substance to be examined with respect to the dissolution behaviour is extractable from the measuring vessel (8), when the movable means is in the position of the test stand (13), the suction pipe (10) therein being liftable and lowerable together with the lifting of the rinsing paddle (17) and/or the paddle (9) relative to the measuring vessel (8),
   **characterized in that**
   - a filter stripping means (22) is arranged at the movable means (6), by means of which (22) the filter (11) can be taken into mesh in a first position of the movable means (6),
   - the filter stripping means (22) is arranged and designed in such manner,
   - during a subsequent lifting of the suction pipe (10) the filter (11) can be stripped off from the suction pipe (10),
   - a filter loading means (25, 26; 28) with receptions for new filters (24) is arranged at the movable means (6), and
   - the suction pipe (10) is lowerable into a second position upon a motion of the movable means (6) and in said lowered position is connectable to the new filter (24) by clamp fit.

2. Dissolution testing apparatus as defined in claim 1,
   **characterized in that**
   - the filter stripping means (22) is disposed such
     - that at first upon a motion of the movable means (6) across a part of its path from the test stand (13) to the rinsing stand (14) into the first position, the filter (11) can be taken into mesh and
   - during a subsequent lifting of the suction pipe (10) the filter (11) can be stripped off from the suction pipe (10).

3. Dissolution testing apparatus as defined in claim 1 or 2,
   **characterized in that**
   - said filter loading means comprises a rotary disk (28) on which a plurality of new filters (24) is arranged at uniform angular distances, and
   - said rotary disk (28) is turnable by the angular distance upon each deposition of a new filter (24) on
   the suction pipe (10) by means of a driving means (29).

4. Dissolution testing apparatus as defined in claim 3,
   **characterized in that**
   - the driving means is a stepping motor.

5. Dissolution testing apparatus as defined in claim 3 or 4,
   **characterized in that**
   - at least two measuring vessels (8), two paddles (9), two rinsing paddles (17) and two suction pipes (10) each one beside another in a direction perpendicular to the direction of motion of the movable means (6) are provided for, and
   - the rotary disk (28) serves as common filter loading means for two adjacent suction pipes (10) and is arranged such that the lowered suction pipes (10) in the second position of the movable means (6) come into mesh with two new filters (24) being arranged on said common rotary disk (28).

6. Dissolution testing apparatus as defined in one of claims 1 to 4,
   **characterized in that**
   a bridge (4) liftable and lowerable in vertical direction is provided for, the paddle (9) as well as the suction pipe (10) being arranged at said bridge (4).

7. Dissolution testing apparatus as defined in one of claims 1 to 6,
   **characterized in that**
   the movable means (6) includes a rinsing vessel (21) into which the the paddle (9) can be submerged in the first position of the movable means (6).

**Revendications**

1. Dispositif pour tester la dissolution (1),
   - muni d'un poste de rinçage (14) qui comporte au moins une palette de rinçage (17) pouvant être entraînée rotativement et pouvant s'élever et s'abaisser dans la direction verticale par rapport à un récipient de mesure (8),
   - muni d'un poste d'essai (13), qui comprend au moins une palette (9) pouvant être entraînée rotativement et pouvant s'élever et s'abaisser dans la direction verticale par rapport au récipient de mesure (8),
   - muni d'un dispositif (6) qui comporte au moins un récipient de mesure (8), dans lequel on peut procéder à l'agitation d'un liquide au moyen de la palette (9) abaissée, liquide qui contient une matière à examiner du point de vue de son comportement à la dissolution, et dispositif (6) qui peut être déplacé entre le poste de rinçage (14) et le poste d'essai (13) en position élevée de la palette (9) et de la palette de rinçage (17),
   - le dispositif (6) pouvant d'une part être déplacé horizontalement par rapport au(x) récipient(s) de mesure et le poste de rinçage (14) et le poste d'essai (13) d'autre part,
   - et muni d'un tuyau d'aspiration (10) comportant un filtre (11), avec lequel tuyau on peut aspirer le liquide contenant la matière à examiner du point de vue de son comportement à la dissolution hors du récipient de mesure (8), lorsque le dispositif mobile se trouve à l'emplacement du poste d'essai (13), le tuyau d'aspiration (10) pouvant être soulevé et abaissé conjointement avec la levée de la palette de rinçage (17) et/ou de la palette (9) par rapport au récipient de mesure (8),
   caractérisé en ce que :
   - un dispositif racleur de filtre (22) est agencé sur le dispositif mobile (6), avec lequel le filtre (11) peut être mis en prise dans une première position du dispositif mobile (6),
   - le dispositif racleur de filtre (22) est agencé et conçu de telle manière que lors d'un levage consécutif du tube d'aspiration (10), le filtre (11) peut être raclé par le tube d'aspiration (10),
   - un dispositif de positionnement de filtre (25, 26 ; 28) muni de logements pour de nouveaux filtres (24) est agencé sur le dispositif mobile (6), et
   - le tube d'aspiration (10) peut être abaissé après un mouvement du dispositif mobile (6) dans une deuxième position et à l'état abaissé peut être relié avec le nouveau filtre (24) par ajustement pressé.

2. Appareil pour tester la dissolution selon la revendication 1,
   caractérisé en ce que :
   - le dispositif racleur de filtre (22) est agencé de telle manière que,
   . dans un premier temps, après un mouvement du dispositif mobile (6) sur une partie de sa course depuis le poste d'essai (13) vers le poste de rinçage (14), le dispositif (22) peut être mis en prise dans la première position du filtre (11),

. lors d'un levage consécutif du tube d'aspiration (10), le filtre peut être raclé par le tube d'aspiration (10).

3. Appareil pour tester la dissolution selon la revendication 1 ou 2,

caractérisé en ce que, le dispositif de positionnement de filtre est conçu sous forme de disque rotatif (28) sur lequel sont agencés selon des espacements angulaires réguliers plusieurs nouveaux filtres (24), et

en ce que le disque rotatif (28) peut être entraîné en rotation par un dispositif d'entraînement (29) après chaque pose d'un nouveau filtre (24) sur le tube d'aspiration (10) selon l'espacement angulaire.

4. Appareil pour tester la dissolution selon la revendication 3,

caractérisé en ce que, le dispositif d'entraînement est un moteur pas-à-pas.

5. Appareil pour tester la dissolution selon la revendication 3 ou 4, caractérisé en ce que,

au moins deux récipients de mesure (8), deux palettes (9), deux palettes de rinçage (17) et deux tubes d'aspiration (10) sont respectivement agencés en juxtaposition dans une direction perpendiculaire au sens de marche du dispositif mobile (6), et

en ce que le disque rotatif (28) sert de dispositif de positionnement de filtre commun pour deux tubes d'aspiration voisins (10) et est agencé de telle manière que les tubes d'aspiration abaissés (10) dans la deuxième position du dispositif mobile (6) sont en prise avec deux nouveaux filtres (24) qui sont agencés sur ce disque rotatif commun (28).

6. Appareil pour tester la dissolution selon l'une des revendications 1 à 4,

caractérisé en ce qu'il est prévu dans la direction verticale un pont relevable et abaissable (4) sur lequel sont agencés la palette (9) ainsi que le tube d'aspiration (10).

7. Appareil pour tester la dissolution selon l'une des revendications 1 à 6,

caractérisé en ce que le dispositif mobile (6) comporte un récipient de rinçage (21) dans lequel la palette (9) peut plonger dans la première position du dispositif mobile (6).

FIG.1

FIG. 2

FIG. 3

FIG. 4

FIG. 5

EP 0 343 261 B1